Europäisches Patentamt

European Patent Office

Office européen des brevets

(11) Veröffentlichungsnummer: **0 301 507 B1**

(12) **EUROPÄISCHE PATENTSCHRIFT**

(45) Veröffentlichungstag der Patentschrift: **28.12.94**

(51) Int. Cl.⁵: **A61B 5/14**, A61M 1/00

(21) Anmeldenummer: **88112136.2**

(22) Anmeldetag: **27.07.88**

(54) **Unterdruck-Sammelbehälter.**

(30) Priorität: **29.07.87 DE 3725193**

(43) Veröffentlichungstag der Anmeldung:
**01.02.89 Patentblatt 89/05**

(45) Bekanntmachung des Hinweises auf die
Patenterteilung:
**28.12.94 Patentblatt 94/52**

(84) Benannte Vertragsstaaten:
**AT BE CH DE ES FR GB GR IT LI LU NL SE**

(56) Entgegenhaltungen:
**DE-U- 1 948 521**
**DE-U- 8 710 428**
**FR-A- 2 428 823**
**US-A- 3 224 276**
**US-A- 3 730 168**

(73) Patentinhaber: **OMNI MEDICAL LIMITED**
**10 Barley Mow Passage**
**London W4 4PH (GB)**

(72) Erfinder: **Der Erfinder hat auf seine Nennung**
**verzichtet**

(74) Vertreter: **Solf, Alexander, Dr. et al**
**Patentanwälte**
**Dr. Solf & Zapf**
**Candidplatz 15**
**D-81543 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft einen Unterdruck-Sammelbehälter insbesondere eine Vakuum-Saugvorrichtung.

Ein Vakuum-Sammelbehälter zum Absaugen von Körperflüssigkeiten, bestückt mit einem transparenten Meßröhrchen mit einem Rohrkanal, einem offenen, mit dem Behälter zu verbindenden Ende sowie einem geschlossenen Ende, wobei der Rohrkanal zumindest teilweise mit einer Flüssigkeit gefüllt ist und zwischen der Flüssigkeit und dem geschlossenen Ende ein Gas, insbesondere Luft, enthalten ist, so daß sich die Flüssigkeit bei Druckänderungen innerhalb des Behälters unter Volumenänderung des Gases innerhalb des Meßröhrchens bewegt, ist aus der US-PS 4 079 729 bekannt. Der bekannte Behälter zur Blutentnahme weist eine Verschlußkappe auf. Die Verschlußkappe wird durch eine Kanüle durchstochen, nachdem letztere zur Blutentnahme in eine Vene eingestochen wurde. Aufgrund des in dem Sammelbehälter vorhandenen Vakuums erfolgt eine gleichmäßige Blutentnahme. Um bereits vor der Anwendung der Blutentnahmevorrichtung dem Anwender Aufschluß darüber zu geben, ob noch ein zur Blutentnahme hinreichendes Vakuum in dem Sammelbehälter vorhanden ist, ist die oben beschriebene Anzeigevorrichtung innerhalb des Behälters angeordnet. Diese bekannte Anzeigevorrichtung bietet jedoch lediglich eine qualitative Anzeige, d.h. sie gestattet lediglich eine Aussage, ob Unterdruck vorhanden ist oder nicht. Das Meßröhrchen weist zwei voneinander beabstandete Markierungsringe auf, zwischen denen bei hinreichendem Unterdruck die Flüssigkeitssäule angeordnet sein muß. Tritt z.B. durch eine Undichtigkeit ein Vakuumverlust auf, so wandert die Flüssigkeit aus dem Bereich zwischen den Markierungsringen heraus; die Vorrichtung soll in diesem Fall nicht mehr verwendet werden.

Bei Vakuum-Saugvorrichtungen zur Wunddrainage, mit denen über längere Zeit hinweg Körperflüssigkeiten, wie Wundsekrete, aus Wundhöhlen abgesaugt werden sollen, ist jedoch eine möglichst genaue, quantitative Anzeige des restlichen Vakuums erwünscht. Derartige Saugvorrichtungen sollen zudem als Einmal-Produkt möglichst preiswert sein. Eine genaue Kalibrierung der aus der US-PS 4 079 729 bekannten Anzeigevorrichtung ist jedoch schwierig, da bei der Herstellung der Vorrichtung, d.h. beim Einfüllen der Flüssigkeit in das einseitig geschlossene Röhrchen, das Volumen des zwischen der Flüssigkeit und dem geschlossenen Ende des Meßröhrchens angeordneten Gases nicht genau bemessen werden kann. Daher müßte jedes Meßröhrchen gesondert mittels einer Unterdruckvorrichtung kalibriert werden, was sehr aufwendig und teuer wäre, so daß diese bekannte Anzeigevorrichtung zur Verwendung bei Einmal-Produkten ungeeignet ist. Zudem ist hier nachteiligerweise eine nicht-lineare Abhängigkeit der Anzeige vom Druck gewährleistet.

Für den beschriebenen Anwendungsfall für Saugvorrichtungen zur Wunddrainage wurden bisher hauptsächlich Druckanzeiger verwendet, die als Faltenbalg ausgeführt sind. Ein derartiger Druckanzeiger ist z.B. in dem DE-GM 73 36 232 beschrieben. Dieser Druckanzeiger schafft jedoch ebenfalls lediglich eine qualitative Anzeige, da die Druck-Deformations-Kennlinie einer Faltenbalgkonstruktion nicht linear verläuft. Außerdem treten bei der Herstellung von Faltenbälgen derartig große individuelle Unterschiede auf, daß eine Kalibrierung des Meßsystems praktisch unmöglich ist. Abgesehen davon ist auch die vorhandene Meßstrecke zu gering, um eine sichere Druckmessung zu gewährleisten.

Aus diesen Gründen ist auch die aus dem DE-GM 79 02 325 bekannte Kombination eines Faltenbalges mit einem Skalenträger zur quantitativen Druckmessung ungeeignet.

Schließlich ist aus der EP 0061723 eine mechanische Anzeigevorrichtung bekannt, die aus einem innerhalb eines Zylinders verschiebbar geführten Kolben besteht, der mit einer Skala des transparenten Zylinders zusammenwirken soll. Bei dieser Ausbildung treten aufgrund der erforderlichen Abdichtung zwischen dem Kolben und der Zylinderwandung hohe Reibungen auf, so daß die Anzeige aufgrund von Hysterese sehr ungenau ist.

Aus der US-A-3 224 276 ist ein Manometer zum Erfassen von Leckagen bekannt. Dieses bekannte Manometer umfaßt einen Flüssigkeitsvorratsbehälter, der einerseits an eine Luftpumpe und den zu prüfenden Gegenstand und andererseits an ein Meßrohr angeschlossen ist, dessen Innendurchmesser ausgehend vom Vorratsbehälter zum anderen Ende abnimmt, um einen möglichst großen Druckbereich sowie einen bei einer Leckage unter großen Drücken auftretenden Druckverlust erfassen zu können.

Der vorliegenden Erfindung liegt die Aufgabe zugrunde, einen Unterdruck-Sammelbehälter mit einer sehr genauen quantitativen Druckanzeige zu schaffen, der sich aber dennoch durch geringe Kosten auch für Einmal-Produkte eignet.

Erfindungsgemäß wird dies durch einen Unterdruck-Sammelbehälter nach Anspruch 1 erreicht. Vorzugsweise verjüngt sich der Rohrkanal des Meßröhrchens von dem offenen Ende in Richtung des geschlossenen Endes leicht konisch. Hierdurch wird vorteilhafterweise eine lineare Abhängigkeit der Anzeige vom Druck erreicht, so daß eine ebenfalls lineare, gut ablesbare Skala verwendet werden kann.

Nach der Erfindung ist des Meßröhrchen zur Bildung des geschlossenen Endes durch eine Verschlußkappe verschlossen, die im Anschluß an den Rohrkanal des Meßröhrchens eine Vertiefung mit einem definierten Volumen aufweist. Durch diese Ausbildung ist es auf sehr einfache Weise möglich, zwischen der Flüssigkeit und dem geschlossenen Ende des Meßröhrchens stets das gleiche, definierte Gas- bzw. Luftvolumen einzubringen, indem nämlich die Verschlußkappe bei Normaldruck dann auf das Röhrchen aufgesetzt wird, wenn die Flüssigkeit genau mit dem Ende des Röhrchens abschließt. Aufgrund des konstanten Gasvolumens läßt sich einmal rechnerisch oder empirisch eine in einer Druckeinheit, z.B. bar, kalibrierte Skala ermitteln, die dann auf allen herzustellenden Meßröhrchen angebracht wird.

Weitere vorteilhafte Ausgestaltungsmerkmale der Erfindung sind in den Unteransprüchen sowie der folgenden Beschreibung enthalten.

Anhand eines in der Zeichnung dargestellten Ausführungs- und Anwendungsbeispiels soll im folgenden die Erfindung näher erläutert werden. Dabei zeigen:

Fig. 1    einen Vakuum-Sammelbehälter mit einer Anzeigevorrichtung,

Fig. 2    einen gegenüber Fig. 1 vergrößerten Längsschnitt durch die Anzeigevorrichtung und

Fig. 3    einen gegenüber Fig. 2 vergrößerten Längsschnitt durch eine Verschlußkappe der Anzeigevorrichtung.

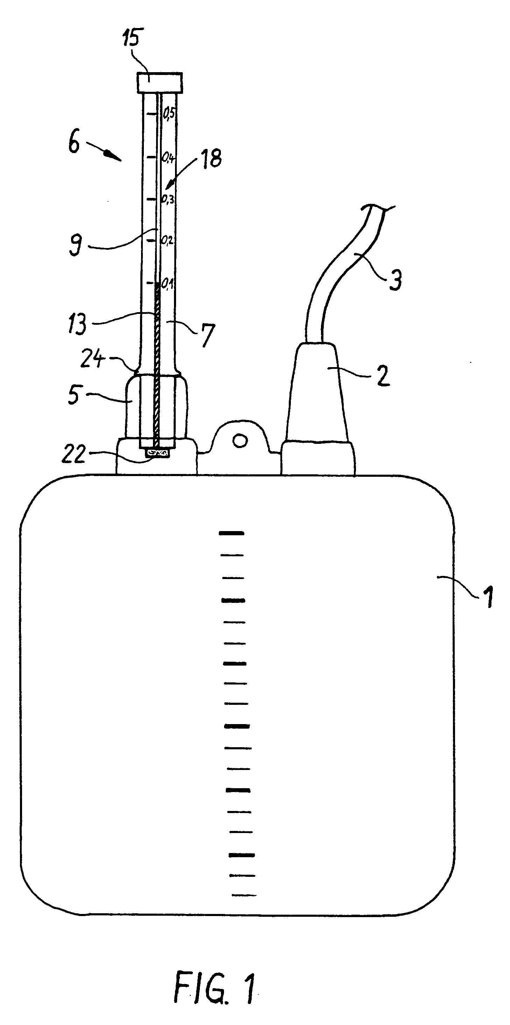

In Fig. 1 ist ein Unterdruck-Sammelbehälter 1 dargestellt, wie er üblicherweise für die Wunddrainage verwendet wird. Der Sammelbehälter 1 weist bereits im Lieferzustand einen Unterdruck von etwa 1/10 des atmosphärischen Druckes auf. Der Behälter 1 hat einen Anschlußstutzen 2 zum druckdichten Einstecken eines Drainageschlauches 3, dessen anderes, nicht dargestelltes Ende z.B. in eine Wundhöhle des Patienten eingeführt wird. Der in dem Behälter 1 vorhandene Unterdruck bewirkt eine rasche und stetige Ableitung von Sekreten, was zu einer Beschleunigung des Heilprozesses führt.

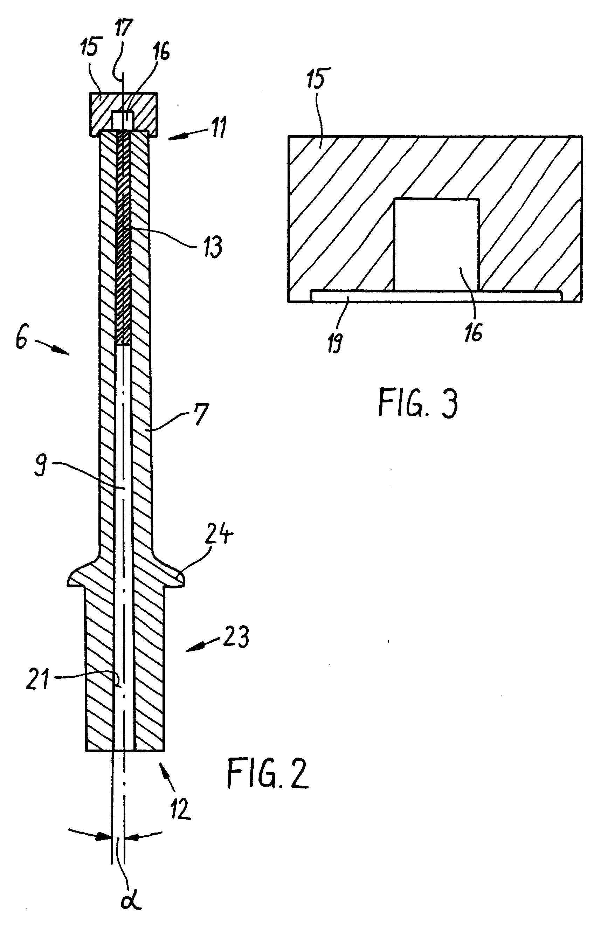

Der Sammelbehälter 1 hat einen weiteren Anschlußstutzen 5, in den die Anzeigevorrichtung 6 dichtend einsteckbar ist. Die Anzeigevorrichtung 6 besteht aus einem Meßröhrchen 7 aus einem hochtransparenten Material und weist einen Rohrkanal 9, ein geschlossenes Ende 11 sowie ein offenes, mit dem Innenraum des Sammelbehälters 1, d.h. mit dem hier vorhandenen Vakuum, zu verbindendes Ende 12 auf. In dem Rohrkanal 9 ist eine vorzugsweise eingefärbte Sperrflüssigkeit 13 sowie zwischen der Flüssigkeit 13 und dem geschlossenen Ende 11 ein Gas, insbesondere Luft, enthalten. Hierdurch bewegt sich die Flüssigkeit 13 bei Druckänderungen innerhalb des Behälters 1 unter Volumenänderung des Gases innerhalb des Rohrkanals 9 des Meßröhrchens 7. In Fig. 1 ist der Zustand dargestellt, in dem durch den in dem Sammelbehälter 1 herrschenden Unterdruck das Gas zwischen dem geschlossenen Ende 11 und der Flüssigkeit 13 expandiert ist, so daß sich die Flüssigkeit 13 zur Anzeige des Unterdruckes (hier 0,1 bar in dem Rohrkanal 9 in Richtung des Behälterinnenraumes bewegt hat. Demgegenüber ist in Fig. 2 das offene Ende 12 mit Normaldruck verbunden.

Erfindungsgemäß ist das Meßröhrchen 7 zur Bildung des geschlossenen Endes 11 durch eine Verschlußkappe 15 verschlossen, die im Anschluß an den Rohrkanal 9 des Meßröhrchens 7 eine Vertiefung 16 mit einem definierten Volumen aufweist. Die Verschlußkappe 15 wird in dem in Fig. 2 dargestellten Zustand, in dem das offene Ende 12 mit atmosphärischem Druck verbunden ist und die Flüssigkeit 13 genau mit dem Ende des Röhrchens 7 abschließt, vorzugsweise von der Seite her, d.h. aus einer zur Röhrchen-Längsachse 17 etwa senkrechten Richtung, aufgesetzt, um Luft bzw. Gas ausschließlich mit dem von der Vertiefung 16 bestimmten Volumen einzuschließen. Aufgrund des definierten Luft- bzw. Gasvolumens kann eine Skala 18, wie z.B. in Fig. 1 dargestellt, ermittelt und auf dem Meßröhrchen 7 angebracht werden.

Die Verschlußkappe 15 (Fig. 3) weist eine die Öffnungsseite der Vertiefung 16 umgebende, in ihrer Flächenform der stirnseitigen Endfläche des Meßröhrchens 7 entsprechende, flache Vertiefung 19 auf. Im Bereich dieser Vertiefung 19 ist das Meßröhrchen 7 mit der Verschlußkappe 15 stoffschlüssig verbunden, z.B. verklebt oder verschweißt.

Weiterhin verjüngt sich der Rohrkanal 9 von dem offenen Ende 12 des Meßröhrchens 7 in Richtung der Verschlußkappe 15 leicht konisch. Vorzugsweise schließt die Innenwandung 21 des Meßröhrchens 7 mit der Röhrchen-Längsachse 17 einen Winkel $\alpha$ von 15' bis 60', vorzugsweise ca. 30' (0,5°) ein (0,25° $\leq \alpha \leq$ 1°). Aufgrund dieser Ausbildung läßt sich die Druck-Skala 18 annähernd linear gestalten. Dabei sind jedoch auch andere, im Sinne der Erfindung gleichwirkende Querschnittsveränderungen des Rohrkanals 7 neben der beschriebenen Konizität möglich.

An dem offenen Ende 12 des Meßröhrchens 7 kann ein hydrophober Filter 22 (Fig. 1) angeordnet sein. Dieser Filter 22 besteht aus einem Material, das für Flüssigkeiten undurchlässig, für Gase jedoch durchlässig ist.

Das Meßröhrchen 7 weist einen sich an das offene Ende 12 anschließenden, vorzugsweise zylindrischen Steckbereich 23 zum Einstecken in den Anschlußstutzen 5 des Sammelbehälters 1 auf. Im Anschluß an den Steckbereich 23 weist das Meßröhrchen 7 auf seiner Außenwandung einen Ringsteg 24 auf, der einen mit dem Anschlußstutzen 5 zusammenwirkenden Einsteck-Begrenzungsanschlag bildet.

Die in dem Meßröhrchen 7 enthaltene Flüssigkeit ist vorzugsweise mit einem Farbstoff, wie Methylenblau B, Patentblau, Kristallviolett, Remazol-Türkisblau B (Firma Hoechst), eingefärbt und somit gut sichtbar. Dabei weist die Flüssigkeit 13 erfindungsgemäß folgende physikalische Eigenschaften auf:

a) niedriger Dampfdruck von $\geq 50°$ C bei 0,05 bar

b) niedrige Löslichkeit von Gasen von nahezu

$$0 \quad \frac{N\ cm^3\ (gas)}{cm^3\ (Lsgm.)\ \cdot\ atm}$$

c) hohe Oberflächenspannung von 10 bis 60 dyn/cm

d) Viskosität von 10 bis 500 cP.

Die Flüssigkeit 13 kann z.B. aus n-Decan, Glyzerin, n-Octan oder Xylol, oder aber aus einem Gemisch dieser Stoffe, bestehen.

Das Meßröhrchen 7 besteht zumindest im Bereich der Skala aus einem hochtransparenten Material, wie z.B. HDPE, PUR, PA oder PEBA. Bei Verwendung eines dieser Materialien treten vorteilhafterweise auch nach Einwirkung von Bestrahlungen, wie z.B. Gamma-Bestrahlung, keine Änderungen der physikalischen Eigenschaften und der Transparenz auf.

Die Skala 18 ist auf dem Meßröhrchen 7 gut sichtbar angebracht, z.B. aufgedruckt, eingeprägt, aufgeklebt oder materialeinheitlich erhaben oder vertieft ausgebildet. Dabei kann es vorteilhaft sein, wenn das Material des Meßröhrchens 7 selbst oder aber ein zusätzlich aufgebrachtes Material ("Schellbachstreifen") einen Vergrößerungs- bzw. Lupeneffekt bewirkt, was die Ablesegenauigkeit der erfindungsgemäßen Anzeigevorrichtung noch erhöht.

Die Anzeigevorrichtung arbeitet nahezu ohne Hysterese, so daß in Verbindung mit der Konizität des Rohrkanals sowie dem definierten Gasvolumen zwischen der Flüssigkeit und der erfindungsgemäßen Verschlußkappe eine sehr genaue, quantitative sowie lageunabhängige Druckanzeige, und zwar sowohl für Unterdrücke von $\leq 0,05$ bar als auch für Überdrücke von $\geq 300$ mm Hg gewährleistet ist.

Dabei wirkt die Flüssigkeit als Sperre bzw. Dichtung für den Druck, so daß auch eine lange Verwendungsmöglichkeit von Unterdruck-Sammelbehältern garantiert werden kann.

**Patentansprüche**

1. Unterdruck-Sammelbehälter (1), insbesondere Vakuum-Saugvorrichtung zum Absaugen von Körperflüssigkeiten, mit einem Anschlußstutzen (2) zum druckdichten Einstecken eines Drainageschlauches (3) und einem weiteren Anschlußstutzen (5) zur Aufnahme einer Anzeigevorrichtung (6) zur Anzeige eines Druckes im Unterdruck-Sammelbehälter (1), die ein transparentes Meßröhrchen (7) mit einem Rohrkanal (9), einem offenen, mit dem Unterdruck-Sammelbehälter (1) verbundenen Ende (12), sowie einem geschlossenen Ende (11) aufweist, wobei der Rohrkanal (9) teilweise mit einer Flüssigkeit (13) gefüllt ist und zwischen der Flüssigkeit (13) und seinem geschlossenen Ende (11) ein definiertes Gasvolumen (16), insbesondere aus Luft, in einer Verschlußkappe (15) zur Bewegbarkeit der Flüssigkeit (13) bei Druckänderungen im Unterdruck-Sammelbehälter (1) unter spezifischer Volumenveränderung des Gases innerhalb des Meßröhrchens (7) vorgesehen ist, und wobei der Rohrkanal (9) im Bereich zwischen dem offenen Ende (12) des Meßröhrchens (7) und dem geschlossenen Ende (11) eine sich verjüngende Querschnittsveränderung aufweist.

2. Unterdruck-Sammelbehälter nach Anspruch 1, **dadurch gekennzeichnet,** daß sich der Rohrkanal (9) vom offenen Ende (12) in Richtung des geschlossenen Endes (11) konisch verjüngt.

3. Unterdruck-Sammelbehälter nach Anspruch 2, **dadurch gekennzeichnet,** daß die Innenwandung (21) des Meßröhrchens (7) mit der Röhrchen-Längsachse (17) einen Winkel ($\alpha$) zwischen 15' und 60', vorzugsweise etwa 30', einschließt.

4. Unterdruck-Sammelbehälter nach einem oder mehreren der Ansprüche 1 bis 3, **dadurch gekennzeichnet,** daß an dem offenen Ende (12) des Meßröhrchens (7) ein hydrophober Filter (22) angeordnet ist, der aus einem flüssigkeitsundurchlässigen und gasdurchlässigen Material besteht.

5. Unterdruck-Sammelbehälter nach einem oder mehreren der Ansprüche 1 bis 4, **dadurch gekennzeichnet,** daß das Meßröhrchen (7) einen sich an das offene Ende (12) anschließenden, vorzugsweise zylindrischen Steckbereich (23) zum Einstecken in den Anschlußstutzen (5) aufweist.

**6.** Unterdruck-Sammelbhälter nach Anspruch 5,
**dadurch gekennzeichnet,**
daß das Meßröhrchen (7) im Anschluß an den Steckbereich (23) auf seiner Außenwandung einen Ringsteg (24) aufweist, der einen mit dem Anschlußstutzen (5) zusammenwirkenden Einsteck-Begrenzungsanschlag bildet.

**7.** Unterdruck-Sammelbehälter nach einem oder mehreren der Ansprüche 1 bis 6,
**dadurch gekennzeichnet,**
daß die Verschlußkappe (15) eine die Öffnungsseite der Vertiefung (16) umgebende, in ihrer Flächenform der Stirnform der stirnseitigen Endfläche des Meßröhrchens (7) enstprechende flache Vertiefung (19) aufweist, in deren Bereich das Meßröhrchen (7) mit der Verschlußkappe (15), vorzugsweise stoffschlüssig verbunden ist.

**8.** Anzeigevorrichtung nach einem oder mehreren der Ansprüche 1 bis 7,
**dadurch gekennzeichnet,**
daß das Meßröhrchen (7) zumindest im Bereich einer Skala (18) aus einem hochtransparenten, bestrahlungs-unempfindlichen Material, insbesondere aus HDPE, PUR, PA oder PEBA, besteht.

**9.** Anzeigevorrichtung nach einem oder mehreren der Ansprüche 1 bis 8,
**dadurch gekennzeichnet,**
daß das Meßröhrchen (7) im Bereich der Skala (18) eine optische Vergrößerungs- bzw. Lupen-Einrichtung aufweist.

**10.** Anzeigevorrichtung nach einem oder mehreren der Ansprüche 1 bis 9,
**dadurch gekennzeichnet,**
daß die in dem Meßröhrchen (7) enthaltene Flüssigkeit (13) aus n-Decan, Glyzerin, n-Octan und/oder Xylol besteht und vorzugsweise Farbstoffe enthält.

**Claims**

**1.** A vacuum collection container (1), in particular a vacuum suction device for sucking up body fluids, with a connecting sleeve (2) for the pressure-tight insertion of a drainage hose (3) and a further connection (5) to receive an indicator device (6) to indicate the pressure in the vacuum collection container (1), which comprises a transparent measuring tube (7) with a duct (9), an open end (12) connected to the vacuum collection container (1) and a closed end (11), wherein the duct (9) is partly filled with a liquid (13), and in which there is provided between the liquid (13) and its closed end (11) a specific volume of gas (16), in particular of air, under a sealing cap (15) for movement of the liquid (13) with pressure changes in the vacuum collection container (1) through specific changes in the volume of the gas within the measuring tube (7), and wherein the duct (9) has a cross-section which tapers between the open end (12) of the measuring tube (7) and the closed end (11).

**2.** A vacuum collection container according to claim 1, characterised in that the duct (9) tapers conically from the open end (12) towards the closed end (11).

**3.** A vacuum collection container according to claim 2, characterised in that the inside wall (21) of the measuring tube (7) makes an angle ($\alpha$) of between 15' and 60', and preferably around 30, with tile longitudinal axis (17) of the tube.

**4.** A vacuum collection container according to one or more of claims 1 to 3, characterised in that a hydrophobic filter (22) made of a liquid- and gas-impermeable material, is located at the open end (12) of the measuring tube (7).

**5.** A vacuum collection container according to one or more of claims 1 to 4, characterised in that the measuring tube (7) has a preferably cylindrical insert portion (23) for insertion into the connection (5) enclosing the open end (12).

**6.** A vacuum collection container according to claim 5, characterised in that the measuring tube (7) has an annular collar (24) on its outside wall adjacent to the insert portion (23), which forms an insertion stop acting in concert with the connection (5).

**7.** A vacuum collection container according to one of more of claims 1 to 6, characterized in that the sealing cap (15) incorporates a flat recess (19) whose shape corresponds to the shape of the end surface of the measuring tube (7) surrounding the open end of recess (16), whereby the measuring tube (7) is attached to the sealing cap (15) preferably by means of a physical seal.

**8.** An indicator device according to one or more of claims 1 to 7, characterised in that the measuring tube (7) consists of a highly transperent radiation-insensitive material, in particular HDPE, PUT, PA or PEBA, at least in the vicinity of a scale (18).

9. An indicator device according to one or more of claims 1 to 8, characterised in that the measuring tube (7) incorporates an optical magnifying or lens device in the area of the scale (18).

10. An indicator device according to one or more of claims 1 to 9, characterised in that the liquid (13) in the measuring tube (7) consists of n-decane, glycerine, n-octane and/or xylol and preferably contains a dye.

**Revendications**

1. Récipient de collecte fonctionnent par dépression (1), en particulier dispositif d'aspiration par le vide pour aspirer des liquides du corps, copmrenant un raccord (2) pour en ficher de façon étanche un tuyau de drainage (3) et un raccord supplémentaire (5) pour recevoir un dispositif d'affichage (6) pour afficher une pression des le récipient de collecte fonctionnent par dépression (1), ledit dispositif d'affichage ayant un tube capilaire gradué (7) transparent avec un canal tubulaire (9), une extrémité (12) ouverte connectée au récipient de collecte fonctionant par dépression (1), ainsi qu'une extrémité fermée (11), le canal tubulaire (9) étant partiellement rempli avec un liquide (13) et un volume de gaz (16), en particulier de l'air, étant prévu entre le liquide (13) et son extrémité fermé (11), dans un capuchon (15) pour la mobilité du liquide (13) lors de variations de pression dans le récipient de collecte fonctionnant par dépression (1) lors d'une variation de volume spécifique du gaz dans le tube capilaire gradué (7), et le canal tubulaire (9) ayant entre l'extrémité ouverte du tube capilaire gradué (7) et l'extrémité fermée d'une variation de section qui diminue.

2. Récipient de collecte fonctionnant par dépression selon la revendication 1, caractérisé en ce que le canal tubulaire (9) diminue de façon conique de l'extrémité ouverte (12) en direction de l'extrémité fermée (11).

3. Récipient de collecte fonctionnant par dépression selon la revendication 2, caractérisé en ce que la paroi intérieure (21) du tube capilaire gradué (17) forme avec l'axe longitudinal (17) du tuyau un angle (α) compris entre 15' et 60', de préférence environ 30'.

4. Récipient de collecte fonctionnant par dépression selon une ou plusieurs des revendications 1 à 3, caractérisé en ce qu'un filtre hydrophobe (22) est arrangé à l'extrémité ouverte (12) du tube capilaire gradué (7), ledit filtre étant constitué par une matière imperméable aux liquides et perméable au gaz.

5. Récipient de collecte fonctionnant par dépression selon une ou plusieurs des revendications 1 à 4, caractérisé en ce que le tube capilaire gradué (7) comprend une zone enfichable (23), de préférence cylindrique suivant l'extrémité ouverte (12) pour être enfichée dans le raccord (5).

6. Récipient de collecte fonctionnant par dépression selon la revendication 5, caractérisé en ce que le tube capilaire gradué (7) comprend à la zone enfichable (23), une nervure circulaire (24) sur sa paroi extérieure, ladite nervure étant formée par un arrêt limiteur d'insertion coopérant avec le raccord (5).

7. Récipient de collecte fonctionnant par dépression selon une ou plusieurs des revendications 1 à 6, caractérisé en ce que le capuchon (15) comprend une cavité (19) plan correspondant, quant à la forme de sa surface, à la forme antérieure de l'extrémité de la face du tube capilaire gradué (7) et entourant le côté ouvert de l'évidement (16), le tube capilaire gradué (7) étant uni de préférence de manière solidaire par la matière, au capuchon (15) dans la zone de la cavité (19).

8. Dispositif d'affichage selon une ou plusieurs des revendications 1 à 7, caractérisé en ce que le tube capilaire gradué (7) est formé, au moins dans la zone de l'échelle (18), d'une matière très transparente, insensible aux radiations, en particulier du HDPE, PUR, PA ou PEBA.

9. Dispositif d'affichage selon une des revendications 1 à 8, caractérisé en ce que le tube capilaire gradué comprend dans la zone de l'échelle (10) un moyen optique d'agrandissement ou de loupe.

10. Dispositif d'affichage selon une des revendications 1 à 9, caractérisé en ce que le liquide (13) contenu dons le tube capilaire gradué (7) est formé de n-décane, glycérine, n-octan et/ou xylol et contient de préférence des matières colorantes.

FIG. 1

FIG. 3

FIG.2